# EUROPÄISCHE PATENTSCHRIFT

(11) **EP 3 272 758 B1**
(45) Veröffentlichungstag und Bekanntmachung des Hinweises auf die Patenterteilung: **27.06.2018**
(21) Anmeldenummer: 16180821.7
(22) Anmeldetag: 22.07.2016
(51) Int. Cl.: C07F 7/18

(54) **VERFAHREN ZUR HERSTELLUNG VON TRIS[3-(ALKOXYSILYL)PROPYL]ISOCYANURATEN**
METHODFOR THE PREPARATION OF TRIS [3- (ALKOXYSILYL) PROPYL] ISOCYANURATES
PROCEDE DE PRODUCTION DE TRI [3-(ALKOXYSILYLE) PROPYL]ISOCYANURATES

(43) Veröffentlichungstag der Anmeldung: 24.01.2018
(73) Patentinhaber: Evonik Degussa GmbH, 45128 Essen (DE)
(72) Erfinder: ALBERT, Philipp, 79618 Rheinfelden (DE); JUST, Eckhard, 79618 Rheinfelden (DE)

(56) Entgegenhaltungen:
- GB-A- 1 202 498
- US-A- 5 986 124
- YOSHINO N ET AL: "Synthesis of bone formation deriving biosilanes", COLLOIDS AND SURFACES. B, BIOINTERFACES, ELSEVIER, AMSTERDAM, NL, Bd. 66, Nr. 1, 25. Mai 2008 (2008-05-25), Seiten 71-76, XP022939862, ISSN: 0927-7765, DOI: 10.1016/J.COLSURFB.2008.05.009 [gefunden am 2008-05-24]

## Beschreibung

Die vorliegende Erfindung betrifft ein besonders wirtschaftliches Verfahren zur Herstellung von Tris[3-(Trialkoxysilyl)propyl]isocyanurat, Tris[3-(Alkyldiialkoxysilyl)propyl]isocyanurat sowie Tris[3-(Dialkylalkoxysilyl)propyl]isocyanurat (nachfolgend in Summe auch kurz Tris[3-(alkoxysilyl)propyl]isocyanurate genannt), wobei man 1,3,5-Triallyl-1,3,5,-triazin-2,4,6(1H,3H,5H9-trion) mit einem Hydrogentrialkoxysilan, Hydrogenalkyldialkoxysilan bzw. Hydrogendialkylalkoxysilan in Gegenwart eines Pt-Ktalysators, einer Carbonsäure und einem weiteren Cokatalysator hydrosilyliert. Tris[3-(alkoxysilyl)propyl]isocyanurat sind Silane, die als Vernetzer eingesetzt werden können. Durch die drei Alkoxysilylgruppen, von der jede Alkoxysilylgruppe nach Hydrolyse eine, zwei bzw. drei chemische Bindungen eingehen kann, sind theoretisch drei bis hin zu neun chemische Bindungen möglich. Durch diese starken Vernetzungsmöglichkeiten sind Tris[3-(alkoxysilyl)propyl]isocyanurate für verschiedene Anwendungen interessant. Ein weiterer Vorteil von Tris[3-(alkoxysilyl)propyl]isocyanuraten ist die hohe Temperaturbeständigkeit, die den Einsatz im Hochtemperaturbereich ermöglicht. Tris[3-(alkoxysilyl)propyl]isocyanurate können daher vorteilhaft als Vernetzer zum Beispiel in Lack- und Kautschukformulierungen sowie als Haftvermittler in Lacken und Klebstoffen in den verschiedensten Industrien eingesetzt werden. Durch die hohe Vernetzungsdichte können darüber hinaus kratzfeste Beschichtungen sowie Barriereschichten erzeugt werden.

JP4266400B beschreibt die Herstellung einer aromatischen Silanverbindung durch Hydrosilylierung einer aromatischen Vinylverbindung. Als Katalysator wird ein Platinkomplex in Anwesenheit einer Carbonsäure eingesetzt.

US 5,986,124 betrifft ein Verfahren zur Herstellung einer Silanverbindung durch Hydrosilylierung einer Kohlenstoffdoppelbindung mittels eines Trialkoxyhydrogensilans in Anwesenheit eines Platinkatalysators und einer Carbonsäure. Durch den Einsatz von Platinkatalysatoren mit Carbonsäuren kann bei der Hydrosilylierung zwar ein Umsatz von ca. 80 % erreicht werden, jedoch weisen so erhaltene Rohprodukte noch einen erheblichen Anteil an Verunreinigungen bzw. Nebenprodukte auf

EP 0587462 beschreibt eine Zusammensetzung aus einem ungesättigten Polyorganosiloxan, einem Organowasserstoffpolysiloxan, einer Säure, einer Platinverbindung sowie Additiven, wobei die Komponenten in Wasser emulgiert und zur Trennschichtbehandlung eingesetzt werden. Die Vernetzung über eine Hydrosilylierung erfolgt beim Erwärmen.

EP 0856517 offenbart einen Prozess zur Hydrosilylierung einer ungesättigten Verbindung in Anwesenheit einer Metallverbindung der 8. bis 10. Nebengruppe des Periodensystems der Elemente. Die Hydrosilylierung wird in Anwesenheit eines Beschleunigers durchgeführt.

In EP 1869058 bzw. WO 2006/113182 wird ein Verfahren zur Herstellung von Tris[3-(Trialkoxysilyl)propyl]isocyanurat vorgestellt. Die Herstellung läuft über das Cracken von Silylorganocarbamat in Anwesenheit einer katalytischen Menge eines Carboxylatsalzes.

EP 0583581 lehrt die Herstellung eines Silylorganocarbamats aus einem Aminosilan. Das Silylorganocarbamat wird nachfolgend in Anwesenheit eines "Crack-Katalysators" zum Silylisocyanurat umgesetzt.

EP 1885731 offenbart ein Verfahren zur Herstellung von Isocyanatosilanen und Silylisocynaurat. Die Synthese startet mit einem Silylorganocarbamat. Durch katalytisches Cracken wird das Isocyanatosilan freigesetzt, wobei die Umsetzung des Isocyanatosilans zum Silylisocyanurat in einer Trimerisationsreaktionszone erfolgt.

CA 943544 beschreibt die Herstellung eines Silylorganoisocyanurats aus einem Halogenalkylsilan und einem Metallcyanat in Anwesenheit eines Lösungsmittels. Das Lösemittel sowie das entstehende Salz werden nach der Reaktion abgetrennt.

US 3,607,901 betrifft die Herstellung von Isocyanatosilanen und Isocyanuratosilanen ausgehend von Chloralkyltrialkoxysilanen und einem Metallcyanat.

US 3,517,001 lehrt u.a. die Herstellung von 1,3,5-Tris(trimethoxysilylpropyl)isocynanurat durch Hydrosilylierung von 1,3,5-Tris(allylisocynaurate) mit Trimethoxysilan in Anwesenheit von Hexachloroplatinsäure. Die Ausbeute wird mit 40 % angegeben.

US 3,821,218 beschreibt die Herstellung von 1,3,5-Tris(trimethoxysilylpropyl)isocyanurat ausgehend Chlorpropyltrimethoxysilansilan und Kaliumcyanat in DMF als Lösungsmittel.

US 2013/0158281 offenbart einen Prozess zur Hydrosilylierung einer ungesättigten Verbindung mit einem Silylhydrid. Als Katalysator werden Fe-, Ni-, Mn- oder Co-Komplexe verwendet.

CN 101805366 beschreibt die Herstellung von 1,3,5-Tris(trimethoxysilylpropyl)isocyanurat durch Cyclokondensation von Isocyanatopropyltrimethoxysilan.

CS 195549 betrifft die Hydrosilylierung von Vinylcyclohexan mit Hydrogensilanen. Dabei wird in Beispiel 4 Vinylcyclohexan mittels Triethoxysilan in Gegenwart von Platinsäure und Trifluoressigsäure hydrosilyliert. Colloids and Surfaces,B, Biointerfaces, 66(1),2003,71-76 bescreibt ein ähnliches Verfahren zur Hydrosilylierung ohne Alkohol und Carbonsäure. Der vorliegenden Erfindung lag die Aufgabe zugrunde, ein Verfahren zur Herstellung von Tris[3-(alkoxysilyl)propyl]isocyanuraten, d.h. aus der Reihe Tris[3-(Trialkoxysilyl)propyl]isocyanurat, Tris[3-(Alkyldiialkoxysilyl)propyl]isocyanurat sowie Tris[3-(Dialkylalkoxysilyl)propyl]isocyanurat, wobei Alkyl insbesondere -aber nicht ausschließlich- für Methyl oder Ethyl sowie Alkoxy für Methoxy oder Ethoxy stehen, bereitzustellen, bei dem ein Pt-Katalysator in Verbindung mit einer Carbonsäure eingesetzt und man die zuvor aufgezeigten Nachteile gegebenenfalls durch eine gezielte Reaktionsführung, gezielte Einsatzstoffverhältnisse und/oder weiterer Zusätze mindert. Darüber hinaus bestanden auch die Anliegen, nach Möglichkeit das Verfahren mit einer möglichst geringen Konzentration an teurem Platin sowie ohne den gesonderten Zusatz eines aliphatischen oder aromatischen Lösemittels durchzuführen und die Ausbeute an Zielprodukt zu steigern. Ebenfalls möchte man den im Zielprodukt verbleibenden Gehalt an Carbonsäure möglichst gering halten.

Überraschenderweise wurde gefunden, dass wesentlich bessere Ausbeuten an Zielprodukt, d.h. einem Tris[3-(alkoxysilyl)propyl]isocyanurat, erzielt werden, wenn man bei der Durchführung der Hydrosilylierung das Hydrogenalkoxysilan zusammen mit einem Pt-Katalysator vorlegt, das vorgelegte Gemisch erwärmt und anschließend unter Durchmischung 1,3,5-Triallyl-1,3,5,-triazin-2,4,6(1H,3H,5H9-trion) als Olefinkomponente zusammen mit einer Carbonsäure über eine definierte Zeitspanne in die Vorlage dosiert und nachreagieren lässt, wobei vorzugsweise der Zusatz einer definierten Menge eines weiteren Cokatalysators zum 1,3,5-Triallyl-1,3,5,-triazin-2,4,6(1H,3H,5H9-trion)-Carbonsäure-Gemisch in Form mindestens eines Alkohols, vorzugsweise eines C1-C10-Alkohols, beispielsweise -aber nicht ausschließlich- aus der Reihe Benzylalkohol, Diglykolmonomethylether, tert.-Butanol, Ethanol und/oder Methanol, und so die Umsetzung sowie die Selektivität und damit die Ausbeute der Hydrosilylierung deutlich fördert.
Dabei hat sich als besonders vorteilhaft erwiesen, das man das vorliegende Verfahren vorzugsweise mit einem homogen Platin(0)-Komplexkatalysator, insbesondere einem "Karstedt-Katalysator", einem Speyer-Katalysator, Hexachloroplatin(IV)säure, oder einem geträgerten, d.h. heterogen Pt-Katalysator, beispielsweise Pt auf Aktivkohle, durchführt. Darüber hinaus setzt man einen Karstedt-Katalysator bevorzugt als Ptatin(0)-Komptexkatatysator-Lösung ein, insbesondere gelöst in Xylol oder Toluol. Ferner ermöglicht es die vorliegende Verfahrensweise, den Gehalt an Pt-Katalysator bzw. Pt-Verlust zu verringern und so teures Pt einzusparen. Weiter hat sich beim vorliegenden Verfahren als besonders vorteilhaft erwiesen, eine Carbonsäure aus der Reihe Benzoesäure, 3,5-Di-tert-butylbenzoesäure, 3,5-Di-tert-butyl-4-hydroxy-benzoesäure, Propionsäure und/oder Essigsäure einzusetzen.
Ein nach dem vorliegenden Verfahren erhaltenes Produktgemisch arbeitet man geeigneterweise durch Destillation, optional unter vermindertem Druck, auf und gewinnt das gewünschte (Ziel-)Produkt.

Bei Einsatz eines Heterogenkatalysators kann man diesen geeigneterweise vor der Destillation vom Produktgemisch trennen, beispielsweise durch Filtration oder Zentrifugieren, und kann diesen so zurückgewonnen Pt-Katalysator vorteilhaft in den Prozess recyclieren.
So gewinnt man das Zielprodukt als Sumpfprodukt bei der nach der Reaktion sowie ggf. nach der Abtrennung eines Heterogenkatalysators durchgeführten Destillation; das Zielprodukt wird bei der destillativen Aufarbeitung nicht überdestilliert und fällt als farbloses Sumpfprodukt an. Darüber hinaus kann man das beschriebene Verfahren schon wirtschaftlich bei einer vergleichsweise niedrigen Temperatur von 40 - 60 °C durchführen.

Gemäß dem vorliegenden Verfahren können so die Doppelbindungen der hier eingesetzten Olefinkomponente vorteilhaft praktisch vollständig hydrosilyliert werden und es entstehen vorteilhaft nur sehr wenige Nebenprodukte.
Darüber hinaus kann das vorliegende, d.h. erfindungsgemäße Verfahren vorteilhaft ohne einen gesonderten Zusatz eines aliphatischen oder aromatischen Kohlenwasserstoffs als Löse- bzw. Verdünnungsmittel und einem nur geringen Anteil an der (Co-)Katalysatorkomponente Carbonsäure, die im Zielprodukt verbleibt, durchgeführt werden.

Gegenstand der vorliegenden Erfindung ist somit ein Verfahren zur Herstellung eines Tris[3-(alkoxysilyl)propyl]isocyanurats aus der Reihe Tris[3-(Trialkoxysilyl)propyl]isocyanurat, Tris[3-(Alkyldiialkoxysilyl)propyl]isocyanurat und Tris[3-(Dialkylalkoxysilyl)propyl]isocyanurat durch Hydrosilylierung,
indem man
- ein Gemisch aus mindestens einem Hydrogenalkoxysilan aus der Reihe Hydrogentrialkoxysilan, Hydrogenalkyldialkoxysilan, Hydrogendialkylalkoxysilan [kurz H-Silan(e) genannt] und einem Pt-Katalysator vorlegt,
- das Gemisch auf eine Temperatur von 40 bis 170 °C erwärmt,
- anschließend unter Durchmischung 1,3,5-Triallyl-1,3,5,-triazin-2,4,6(1H,3H,5H9-trion), mindestens eine Carbonsäure und mindestens einen Alkohol als Cokatalysator, vorzugsweise ein Gemisch enthaltend 1,3,5-Triallyl-1,3,5,-triazin-2,4,6(1H,3H,5H9-trion), mindestens eine Carbonsäure und mindestens einen Alkohol, zusetzt bzw. dosiert,
- reagieren lässt und anschließend das so erhaltene Produktgemisch aufarbeitet.

Beim erfindungsgemäßen Verfahren setzt man H-Silan zu Olefinkomponente vorteilhaft in einem molaren Verhältnis von 1 zu 0,1 bis 0,5, vorzugsweise von 1 zu 0,2 bis 0,4 ein, insbesondere - um hier stellvertretend und beispielhaft nur einige der möglichen und für den Fachmann aus den vorangehenden bzw. vorliegenden Angaben ersichtlichen bzw. erschließbaren Zwischenwerte zu nennen - 1 : 0,13, 1 : 0,15, 1 : 0,18, 1 : 0,23, 1 : 0,25, 1 : 0,28, 1 : 0,3, 1 : 0,33, 1 : 0,35, 1 : 0,38.

Dabei setzt man als H-Silan bevorzugt Hydrogentrimethoxysilan (TMOS), Hydrogentriethoxysilan (TEOS), Methyldiethoxysilan (DEMS), Methyldimethoxysilan (DMMS), Dimethylethoxysilan (DMES) und/oder Dimethylmethoxysilan (MDMS) ein.

Ferner wird beim erfindungsgemäßen Verfahren als Olefinkomponente 1,3,5-Triallyl-1,3,5,-triazin-2,4,6(1H,3H,5H9-trion) eingesetzt.

Vorteilhaft bevorzugt man beim erfindungsgemäßen Verfahren den Einsatz von H-Silan zu Alkohol in einem molaren Verhältnis von 1 zu 0,01 bis 0,2, vorzugsweise 1 zu 0,02 bis 0,18, besonders vorzugsweise 1 zu 0,03 bis 0,15, ganz besonders vorzugsweise 1 zu 0,04 bis 0,1, insbesondere 1 zu 0,05 bis 0,06. Vorzugsweise wählt man dazu mindestens einen Alkohol aus der Reihe der C1-C10-Alkohole aus, besonders vorzugsweise mindestens einen aus der Reihe tert.-Butanol, Ethanol, Methanol, Benzylalkohol sowie Diglykolmonomethylether.

Ferner setzt man beim erfindungsgemäßen Verfahren H-Silan zu Pt vorteilhaft in einem molaren Verhältnis von 1 zu 1 x10⁻⁴ bis 1 x10⁻⁹, vorzugsweise 1 zu 1x10⁻⁵ bis 1 x10⁻⁸, insbesondere von 1 zu 1x10⁻⁵ bis 9 x10⁻⁶, ein.

Dabei verwendet man als Pt-Katalysator geeigneterweise einen Pt-Heterogenkatalysator, vorzugsweise Pt aufgebracht auf einem festen Katalysatorträger, insbesondere Pt auf Aktivkohle, oder einen Pt-Homogenkatalysator, vorzugsweise einen Pt-Komplexkatalysator, wie Hexachloroplatin(IV)säure, auch "Speyer-Katalysator" genannt, insbesondere Hexachloroplatin(IV)säure gelöst in Aceton, vorzugsweise einen Pt(0)-Komplexkatalysator, besonders vorzugsweise einen Karstedt-Katalystor, ganz besonders vorzugsweise einen Platin(0)-1,3-Divinyl-1,1,3,3-Tetramethyldisiloxan-Komplex, insbesondere einen Karstedt-Katalysator in Xylol oder Toluol mit einem Gehalt an Pt(0) von 0,5 bis 5 Gew.-%. Eine solche Lösung enthält in der Regel einen Platin(0) 3-Divinyl-1,1,3,3-Tetramethyldisiloxan Komplex gelöst in Xylol oder Toluol, wobei man eine erfindungsgemäß verwendete Lösung vorteilhaft in verdünnter Form einsetzt und diese vorzugsweise einen Pt-Gehalt von 0,5 bis 5 Gew.-% aufweist. So setzt man beim erfindungsgemäßen Verfahren vorteilhaft einen Pt-Katalysator aus der Reihe Karstedt-Katalysator, insbesondere eine Karstedt-Katalysator-Lösung, vorzugsweise Platin(0)-1,3-Divinyl-1,1,3,3-Tetramethyldisiloxan-Komplex in Xylol oder Toluol mit einem Gehalt an Pt(0) von 0,5 bis 5 Gew.-%, Hexachloroplatin(IV)säure, vorzugsweise Speyer-Katalysator, insbesondere Hexachloroplatin(IV)säure gelöst in Aceton, oder Pt geträgert auf Aktivkohle ein.

Weiter setzt man erfindungsgemäßen Verfahren H-Silan zu Carbonsäure vorzugsweise in einem molaren Verhältnis von 1 zu 1 x10⁻³ bis 30 x10⁻³, besonders vorzugsweise 1 zu 1 x10⁻³ bis 10 x10⁻³, insbesondere von 1 zu 2 x10⁻³ bis 6 x10⁻³, ein.

Dazu wählt man die Carbonsäure bevorzugt aus der Reihe Benzoesäure, 3,5-Di-tert-butylbenzoesäure, 3,5-Di-tert-butyl-4-hydroxy-benzoesäure, Propionsäure, Essigsäure aus.

Bevorzugt führt man das erfindungsgemäße Verfahren aus indem man
- die Komponenten H-Silan und Pt-Katalysator als Gemisch vorlegt und erwärmt,
- Olefinkomponente, Carbonsäure und Alkohol zusammenbringt und das Gemisch enthaltend Olefinkomponente [1,3,5-Triallyl-1,3,5,-triazin-2,4,6(1H,3H,5H9-trion)], Carbonsäure und Alkohol bei einer Temperatur in der Vorlage von 40 - 135 °C unter Durchmischung über eine Zeitspanne von 1 bis 10 Stunden in die Vorlage dosiert und
- nachfolgend über eine Zeitspanne von 0,5 bis 2 Stunden nachreagieren lässt, vorzugsweise führt man die Umsetzung unter Schutzgas durch, insbesondere unter Stickstoff, optional kann man -sofern ein Heterogenkatalysator vorliegt- diesen aus dem so erhaltenen Produktgemisch entfernen,
- die anschließende destillative Aufarbeitung des erhaltenen Produktgemischs führt man bevorzugt bei 45 - 150 °C und einem verminderten Druck aus, wobei man insbesondere vorliegende Tiefsieder, beispielsweise Xylol bzw. Toluol, Alkohol, Carbonsäure, überschüssiges H-Silan bzw. ggf. Olefinkomponente, aus dem Produktgemisch entfernt und das (Ziel-) Produkt gewinnt.

So kann man im Allgemeinen das erfindungsgemäße Verfahren -mit all seinen Kombinationsmöglichkeiten der in der vorliegenden Beschreibung dargelegten Merkmale- wie folgt ausführen:
Für die Durchführung der erfindungsgemäßen Hydrosilylierung zur Herstellung eines Tris[3-(alkoxysilyl)propyl]isocyanurats legt man das Hydrogenalkoxysilan (H-Silan), vorzugsweise Trimethoxysilan (TMOS), Triethoxysilan (TEOS-H), Methyldiethoxysilan (DEMS), Methyldimethoxysilan (DMMS), Dimethylethoxysilan (DMES) oder Dimethylmethoxysilan (MDMS), zusammen mit einem Platin-Katalysator, geeigneterweise einem "Speyer-Katalysator", vorzugsweise Hexachloroplatin(IV)säure in Aceton bzw. Hexachloroplatin(IV)säure-Hexahydrat in Aceton gelöst) oder einem "Karstedt-Katalysator", wobei dieser bevorzugt als Platin(0)-Komplexkatalysator-Lösung eingesetzt wird, oder Pt auf A-Kohle in einem Rührreaktor mit Dosier-, Heiz-/Kühl-, Rückfluss- sowie Destillationsvorrichtung vor, geeigneterweise unter Schutzgas, beispielsweise Stickstoff, und erwärmt das vorgelegte Gemisch auf eine Temperatur von 40 bis 170 °C. Anschließend gibt man unter Durchmischung 1,3,5-Triallyl-1,3,5,-triazin-2,4,6(1H,3H,5H9-trion) als Olefinkomponente, mindestens eine Carbonsäure, vorzugsweise Benzoesäure, 3,5-Di-tert-butylbenzoesäure, 3,5-Di-tert-butyl-4-hydroxy-benzoesäure und/oder Essigsäure, und mindestens einen Alkohol, wie zum Beispiel einem der zuvor genannten C1-C10-Alkohole, als weiteren so genannten Co-Katalysator zu bzw. dosiert: Dabei kann man die Komponenten Olefin, Carbonsäure und Alkohol einzeln nacheinander oder vorteilhaft als 1 ,3,5-Triallyl-1 ,3,5,-triazin-2,4,6(1H,3H,5H9-trion)-Carbonsäure-Alkohol-Gemisch über eine definierte Zeitspanne, vorzugsweise unter Temperaturkontrolle und über 1 bis 10 oder mehr Stunden, wobei die Dosierzeit freilich von der Ansatzgröße und dem Reaktordesign abhängig sein kann, in das Hydrogenalkoxysilan-/Platin-Katalysator-Gemisch der Vorlage portionsweise oder kontinuierlich zusetzen bzw. dosieren und lässt das Reaktions- bzw. Produktgemisch nachreagieren, vorzugsweise unter Durchmischung und Temperaturkontrolle, geeigneterweise bei einer Temperatur von 60 - 100 °C, insbesondere über 0,5 bis 2 Stunden. So setzt man bei vorliegenden Verfahren die jeweiligen Einsatzstoffe bevorzugt einem wohl definierten molaren Verhältnis ein:
- H-Silan zu Olefinkomponente in einem molaren Verhältnis von 1 zu 0,1 bis 0,5
- H-Silan zu Alkohol in einem molaren Verhältnis von 1 zu 0,01 bis 0,2
- H-Silan zu Pt in einem molaren Verhältnis von 1 zu 1 x10⁻⁴ bis 1 x10⁻⁹
- H-Silan zu Carbonsäure in einem molaren Verhältnis von 1 zu 1 x10⁻³ bis 30 x10⁻³ Ferner stellt man die verwendete Karstedt-Katalysator-Lösung bevorzugt aus einem handelsüblichen so genannten Karstedt-Konzentrat (Platin(0) 1,3-Divinyl-1,1,3,3-Tetramethyldisiloxan Komplex, Platingehalt: 20,37 Gew.-%) her, wobei man das Konzentrat vorzugsweise durch den Zusatz von Xylol bzw. Toluol auf einen Pt-Gehalt von 0,5 bis 5 Gew.-% einstellt.

Ein so erhaltenes Produktgemisch arbeitet man geeigneterweise durch Destillation auf und gewinnt so das gewünschte (Ziel-)Produkt. Dazu führt man die Destillation bevorzugt beginnend bei 45 °C bis 150 °C und einem verminderten Druck (Vakuumdestillation bei kleiner 1 bar fallend, insbesondere kleiner gleich 0,1 bar) durch, wobei insbesondere vorliegende Tiefsieder, beispielsweise Carbonsäure, Alkohol, überschüssiges H-Silan bzw. ggf. noch vorliegende Olefinkomponente aus dem Produktgemisch entfernt werden. Sofern ein Pt-Heterogenkatalysator für die Durchführung des erfindungsgemäßen Verfahrens eingesetzt wird, kann man den Pt-Heterogenkatalysator im Rahmen der Produktaufarbeitung aus dem nach der Umsetzung erhaltenen Produktgemisch, d.h. vor dem Destillationsschritt, abtrennen, beispielsweise durch Filtration oder Zentrifugieren, und vorteilhaft wieder in den Prozess zurückführen.

So können erfindungsgemäß Tris[3-(alkoxysilyl)propyl]isocyanurate in vergleichsweise hoher Ausbeute und Selektivität, d.h. mit nur geringen Anteilen an Nebenprodukten in einfacher und wirtschaftlicher Weise vorteilhaft auch großtechnisch hergestellt werden.

Die nachfolgenden Beispiele erläutern die vorliegende Erfindung zusätzlich, ohne den Gegenstand zu beschränken:

### Beispiele:

### Analytische Methoden:

### NMR-Messungen:

Instrument: Bruker
Frequenz: 500,1 MHz (¹H-NMR)
Scans: 32
Temperatur: 303 K
Lösungsmittel CDCl₃
Standard: 0,5 % TMS (Tetramethylsilan)

Nachfolgend sind Erläuterungen zur Benennung hinsichtlich Zielprodukt und bei der Synthese gebildeter Nebenprodukte bzgl. der vorliegenden ¹H-NMR-Auswertungen am Beispiel der Strukturformel eines Tris[3-(trialkoxysilyl)propyl]isocyanurats aufgeführt. Die Bestimmungen für Selektivitäten bzgl. Tris[3-(methyldialkoxysilyl)propyl]-isocyanurat und Tris[3-(dimethylalkoxysilyl)-propyl]isocyanurat wurden in analoger Weise durchgeführt und sind in den Tabellen zu den Beispielen 6 und 7 dargestellt. 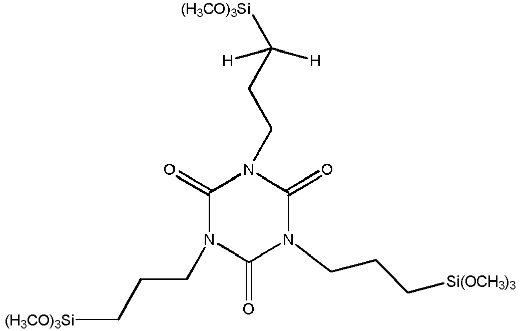 im Zielprodukt: funktionelle Gruppe S1 (Si-CH₂-) 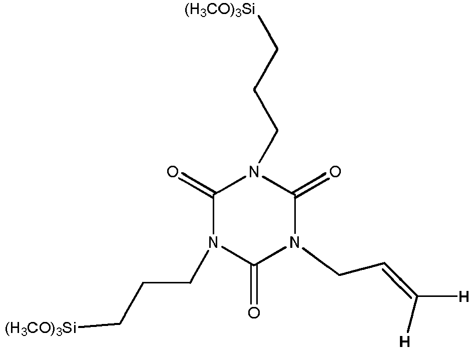 im s.g. Allylderivat: funktionelle Gruppe Al 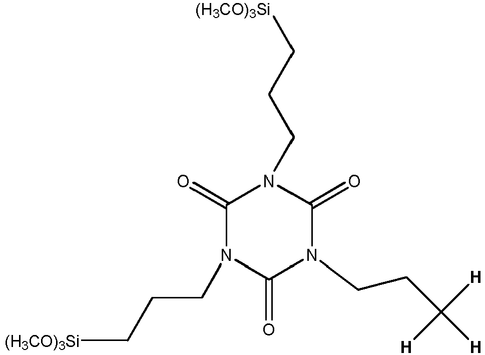 im s.g. Propylderivat: funktionelle Gruppe P1 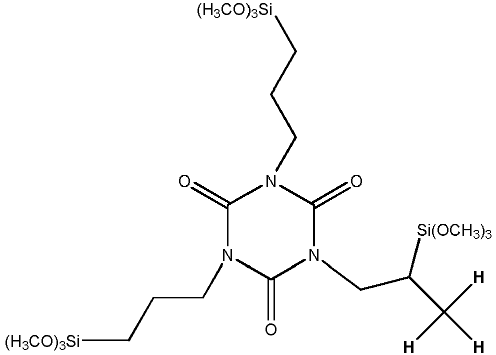 im s.g. Isopropylderivat: funtionelle Gruppe I1

Zur Auswertung der Versuche wurde das bei der Hydrosilylierung am 1,3,5-Triallyl-1,3,5,-triazin-2,4,6(1H,3H,5H9-trion) entstehende Produkt herangezogen. Je mehr allylische Doppelbindungen zum Zielprodukt umgesetzt und je weniger Nebenkomponenten gebildet wurden, desto besser ist die Produktqualität und die Leistungsfähigkeit/Selektivität des Katalysatorsystems. Eine hohe Selektivität ist sehr wichtig, weil die Nebenkomponenten nur mit sehr hohem Aufwand bzw. nicht vom Zielprodukt destillativ entfernt werden können.

Bei der Auswertung der 1H-NMR-Spektren wurden die in den Strukturformeln eingezeichneten Wasserstoffatome herangezogen. Bei der Hydrosilylierung entstehen Si-CH2- Gruppen, die für das Zielprodukt charakteristisch sind. Die Si-CH2- Gruppen wurden mit S1, die allylischen Gruppen (C=CH2-Gruppe) mit A1, die Propylgruppe (-CH3) Gruppe mit P1 und die Isopropylgruppe mit I1 gekennzeichnet. Die Auswertung der 1H-NMR-Spektren und die Berechnung der funktionellen Gruppen wurden nach jedem Versuch in Tabellen dargestellt. Die ausgewerteten Signale aus dem ¹H-NMR bilden für die Gruppe S1 und P1 Triplets (t), für die Gruppe A1 Doppel-Dublets (dd) und für die Gruppe I1 Dublets (d).

### Verwendete Chemikalien:

Karstedt Konzentrat (Platin(0) 1,3-Divinyl-1,1,3,3-Tetramethyldisiloxan Komplex,
Platingehalt: 20,37 Gew.-%), HERAEUS
Aceton, rein, LABC Labortechnik
Hexachloroplatin(IV)säure-hexahydrat, Platingehalt 40 Gew.-%, HERAEUS
Platin-Aktivkohle, Hydrierkatalysator, Platingehalt 10 Gew.-%, MERCK
Benzylalkohol, puriss, SIGMA ALDRICH
Diethylenglykolmonomethylether > 98 Gew.-%, MERCK
Xylol Technical, VWR Chemicals
Dynasylan® TMOS (Trimethoxysilan), EVONIK Industries
Dynasylan® TEOS-H (Triethoxysilan), EVONIK Industries
Dynasylan® DEMS (Methyldiethoxysilan), EVONIK Industries
Dynasylan® DMES (Dimethylethoxysilan), EVONIK Industries
TIACROS® (1,3,5-Triallyl-1,3,5,-triazin-2,4,6(1H,3H,5H9-trion), EVONIK-Industries
Benzoesäure ≥ 99,5 Gew.-%, ROTH
3,5-Di-tert-butylbenzoesäure >98,0 Gew.-%, TOKYO CHEMICAL INDUSTRY
3,5-Di-tert-butyl-4-hydroxybenzoesäure, > Gew.-98,0 %, TOKYO CHEMICAL INDUSTRY
Essigsäure, ≥ 99 Gew.-%, SIGMA-ALDRICH
Methanol ≥ 99,5 Gew.-%, MERCK
Ethanol ≥ 99,8 Gew.-%, ROTH
tert.-Butanol, ≥ 99,0 Gew.-% (zur Synthese), ROTH
Chloroform-d1 (CDCl₃) + 0,5 Gew.-% TMS, DEUTERO
Benzol-d6, DEUTERO
Tetramethylsilan, DEUTERO

### Herstellung Karstedt Katalysator Nr.1 mit 2 Gew.-% Platin Gehalt in Xylol:

In einer 0,2 l Glasflasche wurden 9,8 g Karstedt Konzentrat (Platin(0) 1,3-Divinyl-1,1,3,3-Tetramethyldisiloxan Komplex, Platin Gehalt 20,37 %) mit 90,2 g Xylol vermischt.

### Herstellung Karstedt Katalysator Nr. 2 mit 2 Gew.-% Platin Gehalt in Toluol:

In einer 0,2 l Glasflasche wurden 9,8 g Karstedt Konzentrat (Platin(0) 1,3-Divinyl-1,1,3,3-Tetramethyldisiloxan Komplex, Platin Gehalt 20,37 Gew.-%) mit 90,2 g Toluol vermischt.

### Herstellung Karstedt Katalysator Nr. 3 mit 0,4 Gew.-% Platin Gehalt:

In einer 0,1 l Glasflasche wurden 196,4 mg Karstedt Konzentrat (Platin(0) 1,3-Divinyl-1,1,3,3-Tetramethyldisiloxan Komplex, Platin Gehalt 20,37 Gew.-%) mit 9,8 g Toluol vermischt.

### Herstellung Katalysator 4 aus Hexachloroplatin(IV)säurehexahydrat Lösung in Aceton mit 2,34 Gew.-% Pt Gehalt:

In einem 12 l Kunststoffbehälter wurden 530 g H₂PtCl₆x6H₂O in 9,8 l Aceton aufgelöst. Die so hergestellte Katalysatorlösung wurde nach einer Reifung von 8 Wochen eingesetzt.

### Anmerkung zu den nachfolgenden Vergleichsbeispielen:

Die in US 5,986,124 beschriebene Synthese in Ampullen kann nicht im technischen Maßstab durchgeführt werden. Damit die Versuche besser mit den erfindungsgemäßen Beispielen verglichen werden können, wurden die Versuche in einem Rührkessel bzw. Kolben durchgeführt. Darüber hinaus wurden bei den Beispielen in US 5,986,124 andere ungesättigte Verbindungen eingesetzt, sodass ein direkter Vergleich mit der vorliegenden Erfindung nicht möglich wäre; somit wurde in den folgenden Vergleichsbeispielen TAICROS ® eingesetzt.

### Vergleichsbeispiel 1: (in Anlehnung an Beispiel 1 aus US 5,986,124)

0,2003 Mol (24,5 g) Dynasylan® TMOS, 0,1 ml Katalysator Nr.1, weitere 40,0 g Toluol als zusätzliches Löse- bzw. Verdünnungsmittel, 0,0665 Mol (16,6 g) TAICROS® und 0,4 ml Essigsäure wurden in einer 0,25 I-Rührapparatur mit Intensivkühler vorgelegt und 2,5 Stunden in einem auf 53 - 55 °C erwärmten Ölbad gerührt. Dabei wurden 79,9 g unvollständig umgesetztes und farbloses Sumpfprodukt erhalten. Die leicht flüchtigen Komponenten wurden nicht entfernt.

Auswertung des ¹H-NMR Spektrums bzgl. Vergleichsbeispiel 1:

| Lösungsmittel: CDCl₃ + 0,5 % TMS | Signal bei [ppm] | Integral I | N Anzahl der Protonen | I / N | % (Mol) |
|---|---|---|---|---|---|
| S1 | 0,64 | 100,00 | 2 | 50,00 | 45,8 |
| A1 | 5,27 | 117,28 | 2 | 58,64 | 53,7 |
| P1 | 0,93 | 0,81 | 3 | 0,27 | 0,3 |
| I1 | 1,00 | 0,61 | 3 | 0,20 | 0,2 |

| | | | | | |
|---|---|---|---|---|---|
| Ergebnis: 45,8 % der Allylgruppen wurden durch Hydrosilylierung in Trimethoxysilyalkylgruppen (vgl. S1) umgewandelt. 53,7 % der Allylgruppen (A1) sind nicht umgesetzt worden und es entstanden 0,3 % Propyl (P1)- bzw 0,2 % iso-Propylgruppen (I1), die das Produkt verunreinigen. Die Reaktion ist nicht vollständig. | | | | | |

### Vergleichsbeispiel 2: (in Anlehnung an Beispiel 1 aus US 5,986,124)

0,2003 Mol (32,9 g) Dynasylan® TEOS-H, 0,1 ml Katalysator Nr. 3, weitere 40,0 g Toluol als zusätzliches Löse- bzw. Verdünnungsmittel, 0,0665 Mol (16,6 g) TAICROS® und 0,4 ml Essigsäure wurden in einer 0,25 I-Rührapparatur mit Rückflusskühler, vorgelegt und 2,5 Stunden in einem auf 50 - 57 °C erwärmten Ölbad gerührt. Dabei wurden 88,2 g unvollständig umgesetztes und farbloses Sumpfprodukt erhalten. Die leicht flüchtigen Komponenten wurden nicht entfernt.

Auswertung des ¹H-NMR-Spektrums bzgl. Vergleichsbeispiel 2:

| Lösungsmittel: CDCl₃ + 0,5 % TMS | Signal bei [ppm] | Integral I | N Anzahl der Protonen | I / N | % (Mol) |
|---|---|---|---|---|---|
| S1 | 0,64 | 100,00 | 2 | 50,00 | 86,2 |
| A1 | 5,26 | 14,59 | 2 | 7,30 | 12,6 |
| P1 | 0,94 | 0,33 | 3 | 0,33 | 0,6 |
| I1 | 1,06 | 0,35 | 3 | 0,35 | 0,6 |

| | | | | | |
|---|---|---|---|---|---|
| Ergebnis: 86,2 % der Allylgruppen wurden durch Hydrosilylierung in Trimethoxysilyalkylgruppen (vgl. S1) umgewandelt. 12,6 % der Allylgruppen (A1) sind nicht umgesetzt worden und es entstanden 0,6 % Propyl (P1)- bzw 0,6 % iso-Propylgruppen (I1), die das Produkt verunreinigen. Die Reaktion ist nicht vollständig. | | | | | |

### Vergleichsbeispiel 3: (nur mit Essigsäure, ohne Alkoholzusatz)

1,2 Mol DYNASYLAN® TMOS und 0,2 g Katalysator Nr. 1 (entspricht 0,0205 mMol Pt) in einer 0,5 I-Rührapparatur mit Rückflusskühler, Dosiervorrichtung vorgelegt. Bei einer Temperatur von 76 - 91 °C wurde eine Mischung bestehend aus 0,33 Mol TAICROS® und 6,77 mMol Essigsäure innerhalb 1 h dosiert. Danach ließ man das Gemisch noch ca. 1 weitere Stunde bei ca. 87 - 92 °C weiterreagieren. Anschließend wurden 55,0 g Tiefsieder bei 90 - 120 °C und einem Druck von < 0,1 mbar entfernt. Dabei wurden 170,7 g unvollständig umgesetztes farbloses Sumpfprodukt erhalten.

Auswertung des ¹H-NMR-Spektrums bzgl. Vergleichsbeispiel 3:

| Lösungsmittel: CDCl₃ + 0,5 % TMS | Signal bei [ppm] | Integral I | N Anzahl der Protonen | I / N | % (Mol) |
|---|---|---|---|---|---|
| S1 | 0,66 | 100,00 | 2 | 50,00 | 73,8 |
| A1 | 5,25 | 34,79 | 2 | 17,40 | 25,7 |
| P1 | 0,94 | 0,61 | 3 | 0,20 | 0,3 |
| I1 | 1,01 | 0,43 | 3 | 0,14 | 0,2 |

| | | | | | |
|---|---|---|---|---|---|
| Ergebnis: 73,8 % der Allylgruppen wurden durch Hydrosilylierung in Trimethoxysilyalkyllgruppen (vgl. S1) umgewandelt. 25,7 % der Allylgruppen (A1) sind nicht umgesetzt worden und es entstanden 0,3 % Propyl (P1)- bzw. 0,2 % iso-Propylgruppen (I1), die das Produkt verunreinigen. Die Reaktion ist nicht vollständig. | | | | | |

### Vergleichsbeispiel 4:

1,2 Mol Dynasylan® TMOS, 0,2 g Karstedt Katalysator (entspricht 0,0205 mMol Pt), 34,38 mMol Methanol und 6,55 mMol Benzoesäure wurden in einer 0,5 I-Rührapparatur mit Rückflusskühler, Dosiervorrichtung vorgelegt. Bei einer Temperatur von 73 - 82 °C wurden 0,33 Mol TAICROS® innerhalb 1 h Stunde dosiert. Danach ließ man das Gemisch noch 1 Stunde bei 81 °C weiterreagieren. Anschließend wurden 89,5 g Tiefsieder bei 35 - 127 °C und einem Druck von < 0,1 mbar entfernt. Dabei wurde 134,2 g nicht vollständig umgesetztes und farbloses Sumpfprodukt erhalten.

Auswertung des ¹H-NMR-Spektrums aus Vergleichsbeispiel 4:

| Lösungsmittel: CDCl3 + 0,5 % TMS | Signal bei [ppm] | Integral | N Anzahl der Protonen | I / N | % (Mol) |
|---|---|---|---|---|---|
| S1 | 0,66 | 100,00 | 2 | 50,00 | 42,5 |
| A1 | 5,25 | 134,10 | 2 | 67,05 | 57,0 |
| P1 | 0,94 | 1,24 | 3 | 0,41 | 0,4 |
| I1 | 1,01 | 0,36 | 3 | 0,12 | 0,1 |

| | | | | | |
|---|---|---|---|---|---|
| Ergebnis: 42,5 % der Allylgruppen wurden durch Hydrosilylierung mit TMOS in Trimethoxysilylalkylgruppen (vgl. S1) umgewandelt. 57,0 % der Allylgruppen (A1) wurden nicht umgesetzt. Es entstanden 0,4 % Propyl (P1)- bzw. 0,1 % iso-Propylgruppen (I1), die das Produkt verunreinigen. Die Umsetzung der Allylgruppen ist nicht vollständig und es entstanden nur wenige Nebenprodukte | | | | | |

### Vergleichsbeispiel 5:

1,2 Mol Dynasylan® TMOS, 0,2 g Karstedt Katalysator (entspricht 0,0205 mMol Pt) und 34,38 mMol Methanol wurden in einer 0,5 I-Rührapparatur mit Rückflusskühler, Dosiervorrichtung vorgelegt. Bei einer Temperatur von 70 - 87 °C wurde eine Mischung bestehend aus 0,33 Mol TAICROS® und 6,55 mMol Benzoesäure innerhalb 1 h Stunde dosiert. Danach ließ man das Gemisch noch 1 Stunde bei 81 °C weiterreagieren. Anschließend wurden 41,5 g Tiefsieder bei 61 - 121 °C und einem Druck von < 0,1 mbar entfernt. Dabei wurde 183,0 g nicht vollständig umgesetztes und farbloses Sumpfprodukt erhalten.

| Auswertung des 1H-NMR-Spektrums Vergleichsbeispi el 5:Lösungsmittel: CDCl3 + 0,5 % TMS | Signal bei [ppm] | Integral | N Anzahl der Protonen | I / N | % (Mol) |
|---|---|---|---|---|---|
| S1 | 0,66 | 100,0 | 2 | 50,00 | 81,5 |
| A1 | 5,25 | 21,75 | 2 | 10,87 | 17,7 |
| P1 | 0,94 | 1,09 | 3 | 0,36 | 0,6 |
| I1 | 1,01 | 0,38 | 3 | 0,13 | 0,2 |

| | | | | | |
|---|---|---|---|---|---|
| Ergebnis: 81,5 % der Allylgruppen wurden durch Hydrosilylierung mit TMOS in Trimethoxysilylalkylgruppen (vgl. S1) umgewandelt. 17,7 % der Allylgruppen (A1) wurden nicht umgesetzt. Es entstanden 0,6 % Propyl (P1)- bzw. 0,2 % iso-Propylgruppen (I1), die das Produkt verunreinigen. Die Umsetzung der Allylgruppen ist nicht vollständig und es entstanden nur wenige Nebenprodukte | | | | | |

### Vergleichsbeispiel 6:

0,33 Mol (83,1 g) TAICROS®, 0,2 g Katalysator Nr. 1 (entspricht 0,0205 mMol Pt) und 6,79 mMol (1,7 g) 3,5-Di-tert-butyl-4-hydroxy-benzoesäure wurden in einer 0,5 I-Rührapparatur mit Rückflusskühler, Dosiervorrichtung vorgelegt. Bei einer Temperatur von 91 - 111 °C sollten 1,2 Mol (146,6 g) Dynasylan® TMOS dosiert werden. Die Hydrosilylierung ist stark exotherm und nach einer Dosierung von 18 g Dynasylan® TMOS innerhalb von 9 Minuten stieg die Temperatur bereits von 91 auf 97 °C an. Nachdem weitere 72 g Dynasylan® TMOS innerhalb von 27 Minuten dosiert waren und die Temperatur auf 108 °C angestiegen war, konnte bei weiterer Zugabe von Dynasylan® TMOS keine Exothermie festgestellt werden. Das Reaktionsgemisch kühlte innerhalb von wenigen Minuten von 108 auf 89 °C ab. Der Versuch wurde deshalb nach einer Dosierung von insgesamt 90 g Dynasylan® TMOS abgebrochen, d.h. die Reaktion blieb stehen und die Umsetzung blieb bei dieser Verfahrensweise somit entsprechend unvollständig. 68 g Dynasylan® TMOS wurden nicht dosiert.

### Anmerkung:

Die vorliegenden Vergleichsversuche zur Herstellung von Tris[3-(alkoxysilyl)propyl]isocyanuraten durch Hydrosilylierung von 1,3,5-Triallyl-1,3,5,-triazin-2,4,6(1H,3H,5H9-trion (TAICROS®) in Gegenwart eines Pt-Katalysatorsystems aus Pt-Katalysator und Carbonsäure zeigen, dass man einen vergleichsweise geringer Umsatz der Doppelbindung von deutlich unterhalb 90 Mol-% auffindet, wenn man
- ein Gemisch aus H-Silan, Pt-Katalysator, Carbonsäure und TAICROS® einsetzt, erwärmt und so zur Reaktion bringt
- H-Silan und Pt-Katalysator vorlegt, erwärmt und eine Gemisch aus TAICROS® und Carbonsäure zudosiert
- H-Silan, Pt-Katalysator und Alkohol vorlegt, erwärmt und eine Gemisch aus TAICROS® und Carbonsäure zudosiert
- H-Silan, Pt-Katalysator, Carbonsäure und Alkohol vorlegt, erwärmt und TAICROS® zudosiert oder
- TAICROS®, Pt-Katalysator und Carbonsäure vorlegt, erwärmt und H-Silan zudosiert.

### Beispiel 1:

1,2 Mol Dynasylan® TMOS und 0,2 g Katalysator Nr. 1 (entspricht 0,0205 mMol Pt) wurden in einer 0,5 I-Rührapparatur mit Rückflusskühler, Dosiervorrichtung vorgelegt. Bei einer Temperatur von 74 - 92 °C eine Mischung bestehend aus 0,33 Mol TIACROS®, 6,55 mMol Benzoesäure und 34,38 mMol Methanol innerhalb 1 Stunde dosiert. Danach ließ man das Gemisch noch ca. 1 weitere Stunde bei ca. 84 - 96 °C weiterreagieren. Anschließend wurden 22,4 g Tiefsieder bei 83 - 119 °C und einem Druck von < 0,1 mbar entfernt. Dabei wurde 203,5 g vollständig umgesetztes und farbloses Sumpfprodukt erhalten.

Auswertung des ¹H-NMR-Spektrums bzgl. dem erfindungsgemäßen Beispiel 1:

| Lösungsmittel: CDCl₃ + 0,5 % TMS | Signal bei [ppm] | Integral I | N Anzahl der Protonen | I / N | % (Mol) |
|---|---|---|---|---|---|
| S1 | 0,66 | 100,00 | 2 | 50,00 | 98,6 |
| A1 | 5,25 | 0,01 | 2 | 0,01 | < 0,1 |
| P1 | 0,94 | 1,72 | 3 | 0,57 | 1,1 |
| I1 | 1,01 | 0,38 | 3 | 0,13 | 0,3 |

| | | | | | |
|---|---|---|---|---|---|
| Ergebnis: 98,6 % der Allylgruppen wurden durch Hydrosilylierung in Trimethoxysilyalkylgruppen (vgl. S1) umgewandelt. Allylgruppen (A1) sind nicht mehr nachweisbar. Es entstanden 1,1 % Propyl (P1)- bzw 0,3 % iso-Propylgruppen (I1), die das Produkt verunreinigen. Die Umsetzung der Allylgruppen ist vollständig und es entstanden nur wenige Nebenprodukte. | | | | | |

### Beispiel 2:

1,2 Mol Dynasylan® TMOS und 0,2 g Katalysator Nr. 1 (entspricht 0,0205 mMol Pt) wurden in einer 0,5 I-Rührapparatur mit Rückflusskühler, Dosiervorrichtung vorgelegt. Bei einer Temperatur von 62 - 88 °C wurde eine Mischung bestehend aus 0,33 Mol TIACROS®, 6,55 mMol Benzoesäure und 33,73 mMol tert-Butanol innerhalb 1 Stunde dosiert. Danach ließ man das Gemisch noch ca. 1 weitere Stunde bei ca. 79 - 88 °C weiterreagieren. Anschließend wurden 20,5 g Tiefsieder bei 104 - 118 °C und einem Druck von < 0,1 mbar entfernt. Dabei wurde 204,4 g vollständig umgesetztes und farbloses Sumpfprodukt mit erhalten.

Auswertung des ¹H-NMR-Spektrums bzgl. dem erfindungsgemäßen Beispiel 2:

| Lösungsmittel: CDCl₃+ 0,5 % TMS | Signal bei [ppm] | Integral I | N Anzahl der Protonen | I / N | % (Mol) |
|---|---|---|---|---|---|
| S1 | 0,66 | 100,00 | 2 | 50,00 | 98,5 |
| A1 | 5,26 | 0,05 | 2 | 0,03 | < 0,1 |
| P1 | 0,94 | 1,77 | 3 | 0,59 | 1,2 |
| I1 | 1,01 | 0,40 | 3 | 0,13 | 0,3 |

| | | | | | |
|---|---|---|---|---|---|
| Ergebnis: 98,5 % der Allylgruppen wurden durch Hydrosilylierung in Trimethoxysilyalkylgruppen (vgl. S1) umgewandelt. Allylgruppen (A1) sind nicht mehr nachweisbar. Es entstanden 1,2 % Propyl (P1)- bzw 0,3 % iso-Propylgruppen (I1), die das Produkt verunreinigen. Die Umsetzung der Allylgruppen ist vollständig und es entstanden nur wenige Nebenprodukte. | | | | | |

### Beispiel 3:

1,2 Mol Dynasylan® TMOS und 0,2 g Katalysator Nr. 1 (entspricht 0,0205 mMol Pt) wurden in einer 0,5 I-Rührapparatur mit Rückflusskühler, Dosiervorrichtung vorgelegt. Bei einer Temperatur von 72 - 90 °C wurde eine Mischung bestehend aus 0,33 Mol TIACROS®, 6,66 mMol Essigsäure und 34,38 mMol Methanol innerhalb 1 Stunde dosiert. Danach ließ man das Gemisch noch ca. 1 weitere Stunde bei ca. 78 - 88 °C weiterreagieren. Anschließend wurden 20,0 g Tiefsieder bei 83 - 123 °C und einem Druck von < 0,1 mbar entfernt. Dabei wurde 202,9 g vollständig umgesetztes und farbloses Sumpfprodukt erhalten.

Auswertung des ¹H-NMR-Spektrums bzgl. dem erfindungsgemäßen Beispiel 3:

| Lösungsmittel: CDCl₃ + 0,5 % TMS | Signal bei [ppm] | Integral I | N Anzahl der Protonen | I / N | % (Mol) |
|---|---|---|---|---|---|
| S1 | 0,66 | 100,00 | 2 | 50,00 | 98,6 |
| A1 | 5,25 | 0,23 | 2 | 0,12 | 0,2 |
| P1 | 0,94 | 1,45 | 3 | 0,48 | 1,0 |
| I1 | 1,01 | 0,36 | 3 | 0,12 | 0,2 |

| | | | | | |
|---|---|---|---|---|---|
| Ergebnis: 98,6 % der Allylgruppen wurden durch Hydrosilylierung in Trimethoxysilyalkyllgruppen (vgl. S1) umgewandelt. Allylgruppen (A1) sind noch in sehr geringen Mengen nachweisbar. Es entstanden 1,0 % Propyl (P1)- bzw 0,2 % iso-Propylgruppen (I1), die das Produkt verunreinigen. Die Umsetzung der Allylgruppen ist vollständig und es entstanden nur wenige Nebenprodukte. | | | | | |

### Beispiel 4:

3,5 Mol Dynasylan® TEOS-H und 0,6 g Katalysator Nr. 1 (entspricht 0,0615 mMol Pt) wurden in einer 1 I-Rührapparatur mit Rückflusskühler, Dosiervorrichtung vorgelegt. Bei einer Temperatur von 67 - 97 °C wurde eine Mischung bestehend aus 0,973 Mol TIACROS®, 18,83 mMol Benzoesäure und 102,02 mMol Ethanol innerhalb von 2 Stunden dosiert. Danach ließ man das Gemisch noch ca. 1 weitere Stunde bei ca. 97 - 103 °C weiterreagieren. Anschließend wurden 100,6 g Tiefsieder bei °C und einem Druck von < 0,1 mbar entfernt. Dabei wurde 721,2 g vollständig umgesetztes und farbloses Sumpfprodukt erhalten.

Auswertung des ¹H-NMR-Spektrums bzgl. dem erfindungsgemäßen Beispiel 4:

| Lösungsmittel: CDCl3 + 0,5 % TMS | Signal bei [ppm] | Integral I | N Anzahl der Protonen | I / N | % (Mol) |
|---|---|---|---|---|---|
| S1 | 0,64 | 100,00 | 2 | 50,00 | 98,2 |
| A1 | 5,26 | --- | 2 | --- | < 0,1 |
| P1 | 0,94 | 2,10 | 3 | 0,70 | 1,4 |
| I1 | 1,06 | 0,63 | 3 | 0,21 | 0,4 |

| | | | | | |
|---|---|---|---|---|---|
| Ergebnis: 98,2 % der Allylgruppen wurden durch Hydrosilylierung in Triethoxysilyalkyllgruppen (vgl. S1) umgewandelt. Allylgruppen (A1) sind nicht mehr nachweisbar. Es entstanden 1,4 % Propyl (P1)- bzw 0,4 % iso-Propylgruppen (I1), die das Produkt verunreinigen. Die Umsetzung der Allylgruppen ist vollständig und es entstanden nur wenige Nebenprodukte. | | | | | |

### Beispiel 5:

3,5 Mol Dynasylan® TEOS-H und 0,6 g Katalysator Nr. 1 (entspricht 0,0615 mMol Pt) wurden in einer 1 I-Rührapparatur mit Rückflusskühler, Dosiervorrichtung vorgelegt. Bei einer Temperatur von 70 - 99 °C wurde eine Mischung bestehend aus 0,973 Mol TAICROS®, 19,98 mMol Essigsäure und 102,02 mMol Ethanol innerhalb von 2 Stunden dosiert. Danach ließ man das Gemisch noch ca. 1 weitere Stunde bei ca. 90 - 101 °C weiterreagieren. Anschließend wurden 98,4 g Tiefsieder bei 48 - 145 °C und einem Druck von < 0,1 mbar entfernt. Dabei wurde 719,6 g vollständig umgesetztes und farbloses Sumpfprodukt erhalten.

Auswertung des ¹H-NMR-Spektrums bzgl. dem erfindungsgemäßen Beispiel 5:

| Lösungsmittel: CDCl₃ + 0,5 % TMS | Signal bei [ppm] | Integral | N Anzahl der Protonen | I / N | % (Mol) |
|---|---|---|---|---|---|
| S1 | 0,64 | 100,00 | 2 | 50,00 | 98,7 |
| A1 | 5,26 | --- | 2 | --- | < 0,1 |
| P1 | 0,94 | 1,50 | 3 | 0,50 | 1,0 |
| I1 | 1,06 | 0,45 | 3 | 0,15 | 0,3 |

| | | | | | |
|---|---|---|---|---|---|
| Ergebnis: 98,7 % der Allylgruppen wurden durch Hydrosilylierung in Triethoxysilyalkyllgruppen (vgl. S1) umgewandelt. Allylgruppen (A1) sind nicht mehr nachweisbar. Es entstanden 1,0 % Propyl (P1)- bzw 0,3 % iso-Propylgruppen (I1), die das Produkt verunreinigen. Die Umsetzung der Allylgruppen ist vollständig und es entstanden nur wenige Nebenprodukte. | | | | | |

### Beispiel 6:

0,60 Mol Dynasylan® DEMS (Methyldiethoxysilan) und 0,1 g Katalysator Nr. 1 (entspricht 0,01025 mMol Pt) wurden in einer 0,25 I-Rührapparatur mit Rückflusskühler, Dosiervorrichtung vorgelegt. Bei einer Temperatur von 89 - 125 °C wurde eine Mischung bestehend aus 0,167 Mol TAICROS®, 1,64 mMol Benzoesäure und 32,6 mMol Ethanol innerhalb von 1,5 Stunden dosiert. Danach ließ man das Gemisch noch ca. 1 weitere Stunde bei ca. 110 °C weiterreagieren. Anschließend wurden 12,6 g Tiefsieder bei 65 - 112 °C und einem Druck von < 0,1 mbar entfernt. Dabei wurde 108,4 g vollständig umgesetztes und farbloses Sumpfprodukt erhalten. Neben dem Zielprodukt wurden folgende Spurenverunreinigungen über die ¹H-NMR-Spektren ausgewertet:

Auswertung des ¹H-NMR-Spektrums bzgl. dem erfindungsgemäßen Beispiel 6:

| Lösungsmittel: C₆D₆ + 0,5 % TMS | Signal bei [ppm] | Integral | N Anzahl der Protonen | I / N | % (Mol) |
|---|---|---|---|---|---|
| S1 | 0,62 | 100,00 | 2 | 50,00 | 97,9 |
| A1 | 5,25 | --- | 2 | --- | < 0,1 |
| P1 | 0,75 | 2,33 | 3 | 0,78 | 1,5 |
| I1 | 0,87 | 0,85 | 3 | 0,28 | 0,6 |

| | | | | | |
|---|---|---|---|---|---|
| Ergebnis: 97,9 % der Allylgruppen wurden durch Hydrosilylierung in Methyldiethoxysilyalkylgruppen (vgl. S1) umgewandelt. Allylgruppen (A1) sind nicht mehr nachweisbar. Es entstanden 1,5 % Propyl (P1)- bzw 0,6 % iso-Propylgruppen (I1), die das Produkt verunreinigen. Die Umsetzung der Allylgruppen ist vollständig und es entstanden nur wenige Nebenprodukte. | | | | | |

### Beispiel 7:

0,60 Mol Dynasylan® DMES (Dimethylethoxysilan) und 0,1 g Katalysator Nr. 1 (entspricht 0,01025 mMol Pt) wurden in einer 0,25 I-Rührapparatur mit Rückflusskühler, Dosiervorrichtung vorgelegt. Bei einer Temperatur von 56 - 83 °C wurde eine Mischung bestehend aus 0,167 Mol TAICROS®, 1,64 mMol Benzoesäure und 32,6 mMol Ethanol innerhalb von 1 Stunde dosiert. Danach ließ man das Gemisch noch ca. 1 weitere Stunde bei ca. 88 - 93 °C weiterreagieren. Anschließend wurden 8,8 g Tiefsieder bei 75 - 124 °C und einem Druck von < 0,1 mbar entfernt. Dabei wurde 95,0 g vollständig umgesetztes und farbloses Sumpfprodukt erhalten.

Auswertung des ¹H-NMR-Spektrums bzgl. dem erfindungsgemäßen Beispiel 7:

| Lösungsmittel: C₆D₆ + 0,5 % TMS | Signal bei [ppm] | Integral | N Anzahl der Protonen | I / N | % (Mol) |
|---|---|---|---|---|---|
| S1 | 0,54 | 100,00 | 2 | 50,00 | 99,6 |
| A1 | 5,25 | --- | 2 | --- | < 0,1 |
| P1 | 0,75 | 0,03 | 3 | 0,01 | < 0,1 |
| I1 | 0,87 | 0,57 | 3 | 0,19 | 0,4 |

| | | | | | |
|---|---|---|---|---|---|
| Ergebnis: 99,6 % der Allylgruppen wurden durch Hydrosilylierung in Dimethylethoxysilyalkylgruppen (vgl. S1) umgewandelt. Allylgruppen (A1) und Propylgruppen (P1) sind nicht mehr nachweisbar. Es entstanden 0,4 % iso-Propylgruppen (I1), die das Produkt verunreinigen. Die Umsetzung der Allylgruppen ist vollständig und es entstanden nur wenige Nebenprodukte. | | | | | |

### Beispiel 8:

30,0 Mol Dynasylan® TMOS und 2,4 g Katalysator Nr. 1 (entspricht 0,246 mMol Pt) wurden in einer 8 I-Rührapparatur mit Rückflusskühler, Dosiervorrichtung vorgelegt. Bei einer Temperatur von 74 - 80 °C wurde eine Mischung bestehend aus 9,36 Mol TAICROS®, 184 mMol Benzoesäure und 978 mMol Methanol innerhalb 2 ¾ Stunden dosiert. Danach ließ man das Gemisch noch ca. 1 weitere Stunde bei 80 °C weiterreagieren. Anschließend wurden 264,7 g Tiefsieder bei 140 °C und einem Druck von < 0,1 mbar entfernt. Dabei wurde 5735 g vollständig umgesetztes und farbloses Sumpfprodukt erhalten.

Auswertung des ¹H-NMR-Spektrums bzgl. dem erfindergemäßen Beispiel 8:

| Lösungsmittel: C₆D₆ + 0,5 % TMS | Signal bei [ppm] | Integral I | N Anzahl der Protonen | I / N | % (Mol) |
|---|---|---|---|---|---|
| S1 | 0,66 | 100,0 | 2 | 50,00 | 98,5 |
| A1 | 5,25 | 0,05 | 2 | 0,03 | < 0,1 |
| P1 | 0,94 | 1,83 | 3 | 0,61 | 1,2 |
| I1 | 1,01 | 0,43 | 3 | 0,14 | 0,3 |

| | | | | | |
|---|---|---|---|---|---|
| Ergebnis: 98,5 % der Allylgruppen wurden durch Hydrosilylierung in Trimethoxysilylalkylgruppen (vgl. S1) umgewandelt. Allylgruppen (A1) sind nur noch in sehr geringen Mengen nachweisbar. Es entstanden 1,2 % Propyl (P1)- bzw. 0,2 % iso-Propylgruppen (I1), die das Produkt verunreinigen. Die Umsetzung der Allylgruppen ist vollständig und es entstanden nur wenige Nebenprodukte. | | | | | |

### Beispiel 9:

1,2 Mol Dynasylan® TMOS und 0,2 g Katalysator Nr. 1 (entspricht 0,0205 mMol Pt) wurden in einer 0,5 I-Rührapparatur mit Rückflusskühler, Dosiervorrichtung vorgelegt. Bei einer Temperatur von 45 - 47 °C wurde eine Mischung bestehend aus 0,33 Mol TAICROS®, 6,55 mMol Benzoesäure und 34,38 mMol Methanol innerhalb 2 ¾ Stunden dosiert. Danach ließ man das Gemisch noch 2 ¼ Stunden weiterreagieren, wobei das Reaktionsgemisch langsam exotherm auf 108 °C angestiegen ist. Anschließend wurden 23,3 g Tiefsieder bei 48 - 131 °C und einem Druck von < 0,1 mbar entfernt. Dabei wurde 205,3 g vollständig umgesetztes und farbloses Sumpfprodukt erhalten.

Auswertung des ¹H-NMR-Spektrums bzgl. dem erfindergemäßen Beispiel 9:

| Lösungsmittel: CDCl₃ + 0,5 % TMS | Signal bei [ppm] | Integral | N Anzahl der Protonen | I / N | % (Mol) |
|---|---|---|---|---|---|
| S1 | 0,64 | 100,0 | 2 | 50,00 | 97,9 |
| A1 | 5,26 | --- | 2 | --- | < 0,1 |
| P1 | 0,94 | 1,89 | 3 | 0,63 | 1,2 |
| I1 | 1,06 | 1,34 | 3 | 0,45 | 0,9 |

| | | | | | |
|---|---|---|---|---|---|
| Ergebnis: 97,9 % der Allylgruppen wurden durch Hydrosilylierung mit TMOS in Trimethoxysilylalkylgruppen (vgl. S1) umgewandelt. Allylgruppen (A1) sind nicht mehr nachweisbar. Es entstanden 1,2 % Propyl (P1)- bzw. 0,9 % iso-Propylgruppen (I1), die das Produkt verunreinigen. Die Umsetzung der Allylgruppen ist vollständig und es entstanden nur wenige Nebenprodukte. | | | | | |

### Beispiel 10:

0,9 Mol Dynasylan® TEOS und 0,15 g Katalysator Nr. 1 (entspricht 0,0154 mMol Pt) wurden in einer 0,5 I-Rührapparatur mit Rückflusskühler, Dosiervorrichtung vorgelegt. Bei einer Temperatur von 131 -161 °C wurde eine Mischung bestehend aus 0,25 Mol TAICROS®, 4,83 mMol Benzoesäure und 26,08 mMol Ethanol innerhalb 1 Stunde dosiert. Danach ließ man das Gemisch noch 1 Stunde bei 157 - 162 °C weiterreagieren. Anschließend wurden 21,0 g Tiefsieder bei 78 - 128 °C und einem Druck von < 0,1 mbar entfernt. Dabei wurde 184,3 g vollständig umgesetztes und farbloses Sumpfprodukt erhalten.

Auswertung des ¹H-NMR-Spektrums bzgl. Dem erfindergemäßen Beispiel 10:

| Lösungsmittel: CDCl₃ + 0,5 % TMS | Signal bei [ppm] | Integral | N Anzahl der Protonen | I / N | % (Mol) |
|---|---|---|---|---|---|
| S1 | 0,64 | 100,0 | 2 | 50,00 | 97,5 |
| A1 | 5,26 | 0,03 | 2 | 0,03 | < 0,1 |
| P1 | 0,94 | 0,93 | 3 | 0,93 | 1,8 |
| I1 | 1,06 | 0,34 | 3 | 0,34 | 0,7 |

| | | | | | |
|---|---|---|---|---|---|
| Ergebnis: 97,5 % der Allylgruppen wurden durch Hydrosilylierung mit TMOS in Triethoxysilylalkylgruppen (vgl. S1) umgewandelt. Allylgruppen (A1) sind nur noch in sehr geringen Mengen nachweisbar. Es entstanden 1,8 % Propyl (P1)- bzw. 0,7 % iso-Propylgruppen (I1), die das Produkt verunreinigen. Die Umsetzung der Allylgruppen ist vollständig und es entstanden nur wenige Nebenprodukte. | | | | | |

### Beispiel 11:

3,75 Mol Dynasylan® TMOS und 0,3 g Karstedt Katalysator Nr. 2 (entspricht 0,03845 mMol Pt) wurden in einer 1 I-Rührapparatur mit Rückflusskühler, Dosiervorrichtung vorgelegt. Bei einer Temperatur von 74 - 79 °C wurde eine Mischung bestehend aus 1,17 Mol TAICROS®, 22,93 mMol Benzoesäure und 121,87 mMol Methanol innerhalb 1 Stunde dosiert. Danach ließ man das Gemisch noch ca. 1 weitere Stunde bei 75 - 78 °C weiterreagieren. Anschließend wurden 33,7 g Tiefsieder bei 100 - 129 °C und einem Druck von < 0,1 mbar entfernt. Dabei wurde 716,2 g vollständig umgesetztes und farbloses Sumpfprodukt erhalten.

Auswertung des ¹H-NMR-Spektrums bzgl. Dem erfindergemäßen Beispiel 11:

| Lösungsmittel: CDCl₃ + 0,5 % TMS | Signal bei [ppm] | Integral | N Anzahl der Protonen | I / N | % (Mol) |
|---|---|---|---|---|---|
| S1 | 0,66 | 100,0 | 2 | 50,00 | 98,4 |
| A1 | 5,25 | 0,02 | 2 | 0,01 | < 0,1 |
| P1 | 0,94 | 1,90 | 3 | 0,63 | 1,2 |
| I1 | 1,01 | 0,48 | 3 | 0,16 | 0,3 |

| | | | | | |
|---|---|---|---|---|---|
| Ergebnis: 98,4 % der Allylgruppen wurden durch Hydrosilylierung mit TMOS in Trimethoxysilylalkylgruppen (vgl. S1) umgewandelt. Allylgruppen (A1) sind noch in sehr geringen Mengen nachweisbar. Es entstanden 1,2 % Propyl (P1)- bzw. 0,3 % iso-Propylgruppen (I1), die das Produkt verunreinigen. Die Umsetzung der Allylgruppen ist vollständig und es entstanden nur wenige Nebenprodukte. | | | | | |

### Beispiel 12:

1,2 Mol Dynasylan® TMOS und 0,16 g Katalysator Nr. 4 (entspricht 0,02050 mMol Pt) wurden in einer 0,5 I-Rührapparatur mit Rückflusskühler, Dosiervorrichtung vorgelegt. Bei einer Temperatur von 73 - 84 °C wurde eine Mischung bestehend aus 0,33 Mol TAICROS®, 6,55 mMol Benzoesäure und 34,38 mMol Methanol innerhalb 1 ¼ Stunde dosiert. Danach ließ man das Gemisch noch ca. ¾ Stunden bei 82 - 89 °C weiterreagieren. Anschließend wurden 23,1 g Tiefsieder bei 112 - 125 °C und einem Druck von < 0,1 mbar entfernt. Dabei wurde 204,8 g vollständig umgesetztes und farbloses Sumpfprodukt erhalten.

Auswertung des ¹H-NMR-Spektrums bzgl. Dem erfindergemäßen Beispiel 12:

| Lösungsmittel: CDCl₃ + 0,5 % TMS | Signal bei [ppm] | Integral | N Anzahl der Protonen | I / N | % (Mol) |
|---|---|---|---|---|---|
| S1 | 0,66 | 100,0 | 2 | 50,00 | 98,5 |
| A1 | 5,25 | --- | 2 | --- | < 0,1 |
| P1 | 0,94 | 1,90 | 3 | 0,63 | 1,2 |
| I1 | 1,01 | 0,46 | 3 | 0,30 | 0,3 |

| | | | | | |
|---|---|---|---|---|---|
| Ergebnis: 98,5 % der Allylgruppen wurden durch Hydrosilylierung mit TMOS in Trimethoxysilylalkylgruppen (vgl. S1) umgewandelt. Allylgruppen (A1) sind nicht mehr nachweisbar. Es entstanden 1,2 % Propyl (P1)- bzw. 0,3 % iso-Propylgruppen (I1), die das Produkt verunreinigen. Die Umsetzung der Allylgruppen ist vollständig und es entstanden nur wenige Nebenprodukte. | | | | | |

### Beispiel 13:

1,2 Mol Dynasylan® TMOS und 1,46 g Platin-Aktivkohle (10 % Platin auf Aktivkohle, entspricht 0,748 mMol Pt) wurden in einer 0,5 I-Rührapparatur mit Rückflusskühler, Dosiervorrichtung vorgelegt. Bei einer Temperatur von 72 - 91 °C wurde eine Mischung bestehend aus 0,33 Mol TAICROS®, 6,55 mMol Benzoesäure und 34,38 mMol Methanol innerhalb 1 ¼ Stunde dosiert. Danach ließ man das Gemisch noch ca. ¾ Stunden bei 80 - 89 °C weiterreagieren. Anschließend wurde der Platin-Katalysator über eine Druckfiltration zurückgewonnen und 14,2 g Tiefsieder bei 95 - 138 °C und einem Druck von < 0,1 mbar entfernt. Dabei wurde 198,2 g vollständig umgesetztes und farbloses Sumpfprodukt erhalten.

Auswertung des ¹H-NMR-Spektrums bzgl. Dem erfindergemäßen Beispiel 13:

| Lösungsmittel: CDCl₃ + 0,5 % TMS | Signal bei [ppm] | Integral | N Anzahl der Protonen | I / N | % (Mol) |
|---|---|---|---|---|---|
| S1 | 0,66 | 100,0 | 2 | 50,00 | 98,6 |
| A1 | 5,25 | --- | 2 | --- | < 0,1 |
| P1 | 0,94 | 1,67 | 3 | 0,56 | 1,1 |
| I1 | 1,01 | 0,42 | 3 | 0,14 | 0,3 |

| | | | | | |
|---|---|---|---|---|---|
| Ergebnis: 98,6 % der Allylgruppen wurden durch Hydrosilylierung mit TMOS in Trimethoxysilylalkylgruppen (vgl. S1) umgewandelt. Allylgruppen (A1) sind nicht mehr nachweisbar. Es entstanden 1,1 % Propyl (P1)- bzw. 0,3 % iso-Propylgruppen (I1), die das Produkt verunreinigen. Die Umsetzung der Allylgruppen ist vollständig und es entstanden nur wenige Nebenprodukte. | | | | | |

### Beispiel 14:

1,2 Mol Dynasylan® TMOS und 0,2 g Katalysator Nr. 1 (entspricht 0,0205 mMol Pt) wurden in einer 0,5 I-Rührapparatur mit Rückflusskühler, Dosiervorrichtung vorgelegt. Bei einer Temperatur von 71 - 96 °C wurde eine Mischung bestehend aus 0,33 Mol TAICROS®, 6,55 mMol Benzoesäure und 34,38 mMol Benzylalkohol innerhalb 1 Stunde dosiert. Danach ließ man das Gemisch noch ca. 1 weitere Stunde bei 83 °C weiterreagieren. Anschließend wurden 21,4 g Tiefsieder bei 70 - 136 °C und einem Druck von < 0,1 mbar entfernt. Dabei wurde 207,2 g vollständig umgesetztes und farbloses Sumpfprodukt erhalten.

Auswertung des ¹H-NMR-Spektrums bzgl. Dem erfindergemäßen Beispiel 14:

| Lösungsmittel: CDCl₃ + 0,5 % TMS | Signal bei [ppm] | Integral | N Anzahl der Protonen | I / N | % (Mol) |
|---|---|---|---|---|---|
| S1 | 0,66 | 100,0 | 2 | 50,00 | 98,6 |
| A1 | 5,25 | 0,01 | 2 | < 0,01 | < 0,1 |
| P1 | 0,94 | 1,74 | 3 | 0,58 | 1,1 |
| I1 | 1,01 | 0,42 | 3 | 0,14 | 0,3 |

| | | | | | |
|---|---|---|---|---|---|
| Ergebnis: 98,6 % der Allylgruppen wurden durch Hydrosilylierung mit TMOS in Trimethoxysilylalkylgruppen (vgl. S1) umgewandelt. Allylgruppen (A1) sind noch in sehr geringen Mengen nachweisbar. Es entstanden 1,1 % Propyl (P1)- bzw. 0,3 % iso-Propylgruppen (I1), die das Produkt verunreinigen. Die Umsetzung der Allylgruppen ist vollständig und es entstanden nur wenige Nebenprodukte. | | | | | |

### Beispiel 15:

1,2 Mol Dynasylan® TMOS und 0,2 g Katalysator (entspricht 0,0205 mMol Pt) wurden in einer 0,5 I-Rührapparatur mit Rückflusskühler, Dosiervorrichtung vorgelegt. Bei einer Temperatur von 72 - 90 °C wurde eine Mischung bestehend aus 0,33 Mol TAICROS®, 6,55 mMol Benzoesäure und 34,38 mMol Diethylenglykolmonomethylether innerhalb 1 Stunde dosiert. Danach ließ man das Gemisch noch ca. 1 weitere Stunde bei 84 °C weiterreagieren. Anschließend wurden 22,4 g Tiefsieder bei 92 - 138 °C und einem Druck von < 0,1 mbar entfernt. Dabei wurde 208,7 g vollständig umgesetztes und farbloses Sumpfprodukt erhalten.

Auswertung des ¹H-NMR-Spektrums bzgl. Dem erfindergemäßen Beispiel 15:

| Lösungsmittel: CDCl₃ + 0,5 % TMS | Signal bei [ppm] | Integral | N Anzahl der Protonen | I / N | % (Mol) |
|---|---|---|---|---|---|
| S1 | 0,66 | 100,0 | 2 | 50,00 | 98,5 |
| A1 | 5,25 | 0,03 | 2 | 0,02 | < 0,1 |
| P1 | 0,94 | 1,82 | 3 | 0,61 | 1,2 |
| I1 | 1,01 | 0,40 | 3 | 0,13 | 0,3 |

| | | | | | |
|---|---|---|---|---|---|
| Ergebnis: 98,5 % der Allylgruppen wurden durch Hydrosilylierung mit TMOS in Trimethoxysilylalkylgruppen (S1) umgewandelt. Allylgruppen (A1) sind noch in sehr geringen Mengen nachweisbar. Es entstanden 1,2 % Propyl (P1)- bzw. 0,3 % iso-Propylgruppen (I1), die das Produkt verunreinigen. Die Umsetzung der Allylgruppen ist vollständig und es entstanden nur wenige Nebenprodukte. | | | | | |

## Patentansprüche

1. Verfahren zur Herstellung eines Tris[3-(alkoxysilyl)propyl]isocyanurats aus der Reihe Tris[3-(Trialkoxysilyl)propyl]isocyanurat, Tris[3-(Alkyldiialkoxysilyl)propyl]isocyanurat und Tris[3-(Dialkylalkoxysilyl)propyl]isocyanurat durch Hydrosilylierung,
indem man
- ein Gemisch aus mindestens einem Hydrogenalkoxysilan aus der Reihe Hydrogentrialkoxysilan, Hydrogenalkyldialkoxysilan, Hydrogendialkylalkoxysilan [kurz H-Silan(e) genannt] und einem Pt-Katalysator vorlegt,
- das Gemisch auf eine Temperatur von 40 bis 170 °C erwärmt,
- anschließend unter Durchmischung 1,3,5-Triallyl-1,3,5,-triazin-2,4,6(1H,3H,5H9-trion), mindestens eine Carbonsäure und mindestens einen Alkohol als Cokatalysator zusetzt bzw. dosiert,
- reagieren lässt und anschließend das so erhaltene Produktgemisch aufarbeitet.

2. Verfahren nach Anspruch 1, **dadurch gekennzeichnet,**
**dass** man H-Silan zu Alkohol in einem molaren Verhältnis von 1 zu 0,01 bis 0,2, vorzugsweise 1 zu 0,02 bis 0,18, besonders vorzugsweise 1 zu 0,03 bis 0,15, ganz besonders vorzugsweise 1 zu 0,04 bis 0,1, insbesondere 1 zu 0,05 bis 0,06 einsetzt.

3. Verfahren nach Anspruch 1 oder 2, **dadurch gekennzeichnet,**
**dass** man H-Silan zu Pt in einem molaren Verhältnis von 1 zu 1 x10⁻⁴ bis 1 x10⁻⁹, vorzugsweise 1 zu 1x10⁻⁵ bis 3 x10⁻⁸, insbesondere 1 zu 1x10⁻⁵ bis 9,0 x10⁻⁶, einsetzt.

4. Verfahren nach einem der vorangehenden Ansprüchen, **dadurch gekennzeichnet,**
**dass** man H-Silan zu Carbonsäure in einem molaren Verhältnis von 1 zu 1x10⁻³ bis 30x10⁻³, vorzugsweise 1 zu 2x10³ bis 8x10⁻³, einsetzt.

5. Verfahren nach einem der vorangehenden Ansprüchen, **dadurch gekennzeichnet,**
**dass** man H-Silan zu Olefinkomponente in einem molaren Verhältnis von 1 zu 0,1 bis 0,5, vorzugsweise 1 zu 0,2 bis 0,4, einsetzt.

6. Verfahren nach einem der vorangehenden Ansprüchen, **dadurch gekennzeichnet,**
**dass** man die Carbonsäure aus der Reihe Benzoesäure, Propionsäure, 3,5-Di-tert-butylbenzoesäure, 3,5-Di-tert-butyl-4-hydroxy-benzoesäure, Essigsäure auswählt.

7. Verfahren nach einem der vorangehenden Ansprüchen, **dadurch gekennzeichnet,**
**dass** man den Alkohol aus der Reihe der C1-C10-Alkohole auswählt, vorzugsweise aus der Reihe tert.-Butanol, Ethanol, Methanol, Benzylalkohol sowie Diglykolmonomethylether.

8. Verfahren nach einem der vorangehenden Ansprüchen, **dadurch gekennzeichnet,**
**dass** man als H-Silan Hydrogentrimethoxysilan (TMOS), Hydrogentriethoxysilan (TEOS), Methyldiethoxysilan (DEMS), Methyldimethoxysilan (DMMS), Dimethylethoxysilan (DMES) oder Dimethylmethoxysilan (MDMS) einsetzt.

9. Verfahren nach einem der vorangehenden Ansprüchen, **dadurch gekennzeichnet,**
**dass** man einen Pt-Katalysator aus der Reihe Karstedt-Katalysator, vorzugsweise Platin(0)-1,3-Divinyl-1,1,3,3-Tetramethyldisiloxan-Komplex, insbesondere als Karstedt-Katalysator in Xylol oder Toluol mit einem Gehalt an Pt(0) von 0,5 bis 5 Gew.-%, Hexachloroplatin(IV)säure, vorzugsweise Speyer-Katalysator, insbesondere Hexachloroplatin(IV)säure gelöst in Aceton, oder Pt aufgebracht auf einem festen Katalysatorträger, vorzugsweise Pt geträgert auf Aktivkohle, einsetzt.

10. Verfahren nach einem der vorangehenden Ansprüchen, **dadurch gekennzeichnet,**
**dass** man
- die Komponenten H-Silan und Pt-Katalysator als Gemisch vorlegt und erwärmt,
- Olefinkomponente, Carbonsäure und Alkohol zusammenbringt und das Gemisch enthaltend Olefinkomponente [1,3,5-Triallyl-1,3,5,-triazin-2,4,6(1H,3H,5H9-trion)], Carbonsäure und Alkohol bei einer Temperatur in der Vorlage von 40 - 135 °C unter Durchmischung über eine Zeitspanne von 1 bis 10 Stunden in die Vorlage dosiert und
- nachfolgend über eine Zeitspanne von 0,5 bis 2 Stunden nachreagieren lässt, vorzugsweise führt man die Umsetzung unter Schutzgas durch, insbesondere unter Stickstoff, optional kann man -sofern ein Heterogenkatalysator vorliegt- diesen aus dem so erhaltenen Produktgemisch entfernen,
- die anschließende destillative Aufarbeitung des erhaltenen Produktgemischs führt man bevorzugt bei 45 - 150 °C und einem verminderten Druck aus, wobei man insbesondere vorliegende Tiefsieder, beispielsweise Xylol bzw. Toluol, Alkohol, Carbonsäure, überschüssiges H-Silan bzw. ggf. Olefinkomponente, aus dem Produktgemisch entfernt und das (Ziel-) Produkt gewinnt.

## Claims

1. Process for preparing a tris[3-(alkoxysilyl)propyl] isocyanurate from the group of tris[3-(trialkoxysilyl)propyl] isocyanurate, tris[3-(alkyldialkoxysilyl)propyl] isocyanurate and tris[3-(dialkylalkoxysilyl)propyl] isocyanurate by hydrosilylation,
by
- initially charging a mixture of at least one hydroalkoxysilane from the group of hydrotrialkoxysilane, hydroalkyldialkoxysilane, hydrodialkylalkoxysilane [called H-silane(s) for short] and a Pt catalyst,
- heating the mixture to a temperature of 40 to 170°C,
- then adding or metering in 1,3,5-triallyl-1,3,5-triazine-2,4,6(1H,3H,5H)-trione, at least one carboxylic acid and at least one alcohol as cocatalyst while mixing,
- leaving the mixture to react and then working up the product mixture thus obtained.

2. Process according to Claim 1, **characterized in that**
H-silane is used relative to alcohol in a molar ratio of 1:0.01 to 0.2, preferably 1:0.02 to 0.18, more preferably 1:0.03 to 0.15, even more preferably 1:0.04 to 0.1, especially 1:0.05 to 0.06.

3. Process according to Claim 1 or 2, **characterized in that**
H-silane is used relative to Pt in a molar ratio of 1:1 x 10⁻⁴ to 1 x 10⁻⁹, preferably 1:1 x 10⁻⁵ to 3 x 10⁻⁸, especially 1:1 x 10⁻⁵ to 9.0 x 10⁻⁶.

4. Process according to any of the preceding claims,
**characterized in that**
H-silane is used relative to carboxylic acid in a molar ratio of 1:1 x 10⁻³ to 30 x 10⁻³, preferably 1:2 x 10⁻³ to 8 x 10⁻³.

5. Process according to any of the preceding claims,
**characterized in that**
H-silane is used relative to olefin component in a molar ratio of 1:0.1 to 0.5, preferably 1:0.2 to 0.4.

6. Process according to any of the preceding claims,
**characterized in that**
the carboxylic acid is selected from the group of benzoic acid, propionic acid, 3,5-di-tert-butylbenzoic acid, 3,5-di-tert-butyl-4-hydroxybenzoic acid, acetic acid.

7. Process according to any of the preceding claims,
**characterized in that**
the alcohol is selected from the group of the C1-C10 alcohols, preferably from the group of tert-butanol, ethanol, methanol, benzyl alcohol and diglycol monomethyl ether.

8. Process according to any of the preceding claims,
**characterized in that**
the H-silane used is hydrotrimethoxysilane (TMOS), hydrotriethoxysilane (TEOS), methyldiethoxysilane (DEMS), methyldimethoxysilane (DMMS), dimethylethoxysilane (DMES) or dimethylmethoxysilane (MDMS).

9. Process according to any of the preceding claims,
**characterized in that**
a Pt catalyst from the Karstedt catalyst group is used, preferably platinum(0)-1,3-divinyl-1,1,3,3-tetramethyldisiloxane complex, especially in the form of Karstedt catalyst in xylene or toluene with a Pt(0) content of 0.5% to 5% by weight, hexachloroplatinum(IV) acid, preferably Speyer catalyst, especially hexachloroplatinum(IV) acid dissolved in acetone, or Pt applied to a solid catalyst support, preferably Pt supported on activated carbon.

10. Process according to any of the preceding claims,
**characterized in that**
- the H-silane and Pt catalyst components are initially charged and heated as a mixture,
- olefin component, carboxylic acid and alcohol are combined and the mixture comprising olefin component [1,3,5-triallyl-1,3,5-triazine-2,4,6(1H,3H,5H)-trione], carboxylic acid and alcohol is metered into the initial charge at a temperature in the initial charge of 40-135°C while mixing over a period of time of 1 to 10 hours and
- the mixture is subsequently left to react over a period of time of 0.5 to 2 hours, the reaction preferably being conducted under protective gas, especially under nitrogen; it is optionally possible - if a heterogeneous catalyst is used - to remove it from the product mixture thus obtained,
- the subsequent distillative workup of the product mixture obtained is performed preferably at 45-150°C and a reduced pressure, by removing especially low boilers that are present, for example xylene/toluene, alcohol, carboxylic acid, excess H-silane and optionally olefin component, from the product mixture to obtain the (target) product.

## Revendications

1. Procédé de fabrication d'un isocyanurate de tris[3-(alcoxysilyl)propyle] de la série constituée par l'isocyanurate de tris[3-(trialcoxysilyl)propyle], l'isocyanurate de tris[3-(alkyldialcoxysilyl)propyle] et l'isocyanurate de tris[3-(dialkylalcoxysilyl)propyle] par hydrosilylation, selon lequel
- un mélange d'au moins un hydrogénoalcoxysilane de la série constituée par l'hydrogénotrialcoxysilane, l'hydrogénoalkyldialcoxysilane, l'hydrogénodialkylalcoxysilane [en abrégé nommés H-silane(s)] et d'un catalyseur Pt est chargé,
- le mélange est porté à une température de 40 à 170 °C,
- puis, sous mélange, de la 1,3,5-triallyl-1,3,5-triazine-2,4,6(1H,3H,5H9-trione), au moins un acide carboxylique et au moins un alcool en tant que co-catalyseur sont ajoutés,
- le mélange est laissé réagir, puis le mélange de produits ainsi obtenu est traité.

2. Procédé selon la revendication 1, **caractérisé en ce que** le H-silane et l'alcool sont utilisés en un rapport molaire de 1 sur 0,01 à 0,2, de préférence de 1 sur 0,02 à 0,18, de manière particulièrement préférée de 1 sur 0,03 à 0,15, de manière tout particulièrement préférée de 1 sur 0,04 à 0,1, notamment de 1 sur 0,05 à 0,06.

3. Procédé selon la revendication 1 ou 2, **caractérisé en ce que** le H-silane et le Pt sont utilisés en un rapport molaire de 1 sur 1 x 10⁻⁴ à 1 x 10⁻⁹, de préférence de 1 sur 1 x 10⁻⁵ à 3 x 10⁻⁸, notamment de 1 sur 1 x 10⁻⁵ à 9, 0 x 10⁻⁶.

4. Procédé selon l'une quelconque des revendications précédentes, **caractérisé en ce que** le H-silane et l'acide carboxylique sont utilisés en un rapport molaire de 1 sur 1 x 10⁻³ à 30 x 10⁻³, de préférence de 1 sur 2 x 10⁻³ à 8 x 10⁻³.

5. Procédé selon l'une quelconque des revendications précédentes, **caractérisé en ce que** le H-silane et le composant oléfinique sont utilisés en un rapport molaire de 1 sur 0,1 à 0,5, de préférence de 1 sur 0,2 à 0,4.

6. Procédé selon l'une quelconque des revendications précédentes, **caractérisé en ce que** l'acide carboxylique est choisi dans la série constituée par l'acide benzoïque, l'acide propionique, l'acide 3,5-di-tert.-butylbenzoïque, l'acide 3,5-di-tert.-butyl-4-hydroxybenzoïque, l'acide acétique.

7. Procédé selon l'une quelconque des revendications précédentes, **caractérisé en ce que** l'alcool est choisi dans la série constituée par les alcools en C1-C10, de préférence dans la série constituée par le tert.-butanol, l'éthanol, le méthanol, l'alcool benzylique et l'éther monométhylique de diglycol.

8. Procédé selon l'une quelconque des revendications précédentes, **caractérisé en ce que** de l'hydrogénotriméthoxysilane (TMOS), de l'hydrogénotriéthoxysilane (TEOS), du méthyldiéthoxysilane (DEMS), du méthyldiméthoxysilane (DMMS), du diméthyléthoxysilane (DMES) ou du diméthylméthoxysilane (MDMS) est utilisé en tant que H-silane.

9. Procédé selon l'une quelconque des revendications précédentes, **caractérisé en ce qu'**un catalyseur Pt de la série constituée par un catalyseur de Karstedt, de préférence le complexe platine (0)-1,3-divinyl-1,1,3,3-tétraméthyldisiloxane, notamment sous la forme d'un catalyseur de Karstedt dans du xylène ou du toluène ayant une teneur en Pt(0) de 0,5 à 5 % en poids, l'acide hexachloroplatinique (IV), de préférence le catalyseur de Speyer, notamment l'acide hexachloroplatinique (IV) dissous dans de l'acétone, ou Pt appliqué sur un support de catalyseur solide, de préférence Pt supporté sur du charbon actif, est utilisé.

10. Procédé selon l'une quelconque des revendications précédentes, **caractérisé en ce que**
- les composants H-silane et catalyseur Pt sont chargés sous la forme d'un mélange et chauffés,
- le composant oléfinique, l'acide carboxylique et l'alcool sont rassemblés et le mélange contenant le composant oléfinique [1,3,5-triallyl-1,3,5-triazine-2,4,6(1H,3H,5H9-trione)], l'acide carboxylique et l'alcool est introduit dans le mélange chargé initialement à une température dans le mélange chargé initialement de 40 à 135 °C avec mélange en une durée de 1 à 10 heures, puis
- le mélange est laissé réagir pendant une durée de 0,5 à 2 heures,
la réaction étant de préférence réalisée sous un gaz protecteur, notamment sous de l'azote et, si un catalyseur hétérogène est présent, celui-ci pouvant éventuellement être éliminé du mélange de produits ainsi obtenu,
- le traitement par distillation ultérieur du mélange de produits obtenu est de préférence réalisé à une température de 45 à 150 °C et sous une pression réduite, les composants de faible point d'ébullition présents, par exemple le xylène ou le toluène, l'alcool, l'acide carboxylique, le H-silane en excès et éventuellement le composant oléfinique étant notamment éliminés du mélange de produits et le produit (cible) étant obtenu.
